# EUROPEAN PATENT APPLICATION

(11) **EP 2 745 867 A1**
(43) Date of publication of application: **25.06.2014**
(21) Application number: 12197807.6
(22) Date of filing: 18.12.2012
(51) Int. Cl.: A61M 16/04, A61M 16/08, A61M 25/02, A61M 39/10, F16L 3/04, F16L 3/08, F16L 3/10, F16L 3/12, F16L 3/13, F16L 37/14, F16L 37/18

(54) **Fastening assembly for adjustable tracheotomy tube**

(71) Applicant: Vitaltec Corporation, Taichung City (TW)
(72) Inventor: Chiu, Sheng-Yu, Taichung City (TW); Chang, Ti-Li, Taichung City (TW)
(74) Representative: Pallini Gervasi, Diego

(57) **Abstract**

A fastening assembly for adjustable tracheotomy tube has a plug (10,50), a clamping member (20, 60) and a connector (30,70). The clamping member (20,60) has a sleeve (21,61), a connecting section (22,62) and a tab (23,63). The sleeve (21,61) is slidably mounted in the plug (10,50). The connecting section (22,62) is mounted through the plug (10,50) and the connector (30,70). The tab (23,63) is located outside the plug (10,50) and the connector (30,70) and is pivotally connected with the connecting section (22,62). The tab (23,63) is capable of pressing against the connector (30,70) such that the sleeve (21,61) can be slid in the plug (10,50). It is convenient for a user, especially for taking care of a patient, to pivot the tab (23,63) with one hand. Accordingly, the tracheotomy tube (A) is fastened with ease.

## Description

### 1. Field of the Invention

The present invention relates to a fastening assembly for adjustable tracheotomy tube, and more particularly to a fastening assembly for adjustable tracheotomy tube that is easily used with one hand.

### 2. Description of Related Art

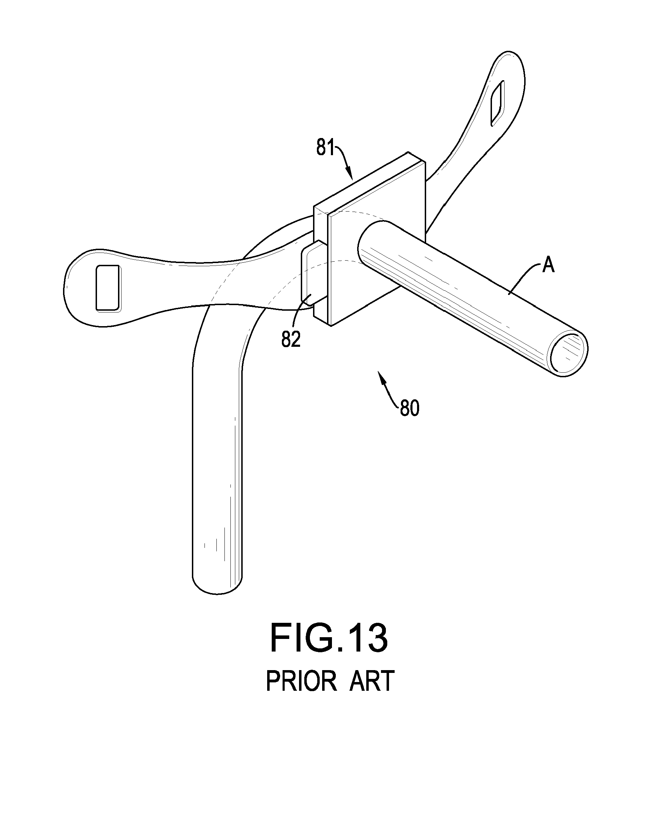

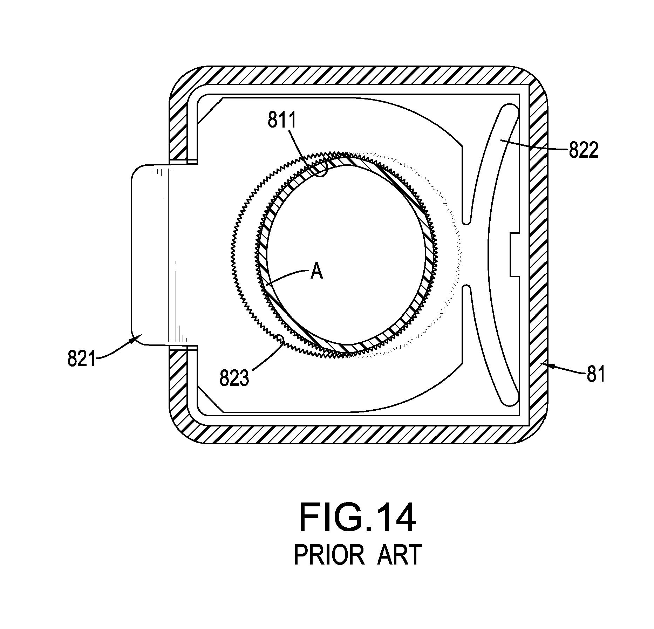

With reference to Figs. 13 and 14, a conventional fastening assembly for adjustable tracheotomy tube 80 has a housing 81 and a tab 82. The housing 81 has a through hole 811. The through hole 811 is formed through the housing 81 and has a jagged inner surface. The tab 82 is movably mounted in the housing 81 and has a pushed section 821, a flexible section 822 and a tab hole 823. The pushed section 821 protrudes out from the housing 81. The flexible section 822 is arcuate, is mounted in the housing 81 and abuts the housing 81. The tab hole 823 is formed through the tab 82, is located between the pushed section 821 and the flexible section 822, and is capable of aligning with the through hole 811 of the housing 81. The tab hole 823 has a jagged inner surface.

When a tracheotomy tube A is about to be inserted into the conventional fastening assembly for adjustable tracheotomy tube 80, first, the pushed section 821 is pushed by a user, and the flexible section 822 deforms to be pressed tightly against the housing 81. Second, the tab hole 823 aligns with the through hole 811, and then the tracheotomy tube A is mounted through the through hole 811 and the tab hole 823. Consequently, the flexible section 822 is released to return to an original position, such that the tracheotomy tube A is fastened.

However, two hands of the user are required to operate the conventional fastening assembly for adjustable tracheotomy tube 80. This is because one hand has to push and hold the tab 82, and the other hand has to hold and insert the tracheotomy tube A. Consequently, this is inconvenient for taking care of a patient because both hands are needed.

Furthermore, the flexible section 822 may lose flexibility due to frequent use, such that the tab 82 can no longer tightly abut the tracheotomy tube A.

To overcome the shortcomings, the present invention tends to provide a fastening assembly for adjustable tracheotomy tube to mitigate the aforementioned problems.

The main objective of the invention is to provide a fastening assembly for adjustable tracheotomy tube that is easily adjusted with one hand.

A fastening assembly for adjustable tracheotomy tube has a plug, a clamping member and a connector. The clamping member has a sleeve, a connecting section and a tab. The sleeve is slidably mounted in the plug. The connecting section is mounted through the plug and the connector. The tab is located outside the plug and the connector and is pivotally connected with the connecting section. The tab is capable of pressing against the connector such that the sleeve can be slid in the plug. It is convenient for a user, especially for taking care of a patient, to pivot the tab with one hand. Accordingly, the tracheotomy tube is fastened with ease.

Other objects, advantages and novel features of the invention will become more apparent from the following detailed description when taken in conjunction with the accompanying drawings.

### IN THE DRAWINGS

Fig. 1 is a perspective view of a first embodiment of a fastening assembly for adjustable tracheotomy tube in accordance with the present invention;
Fig. 2 is an exploded perspective view of the fastening assembly for adjustable tracheotomy tube in Fig. 1;
Fig. 3 is a cross sectional side view of the fastening assembly for adjustable tracheotomy tube in Fig. 2;
Fig. 4 is operational views of the fastening assembly for adjustable tracheotomy tube in Fig. 1, showing that a tracheotomy tube is fastened;
Fig. 5 is a cross sectional top view of the fastening assembly for adjustable tracheotomy tube in Fig. 1;
Fig. 6 is a cross sectional top view of the fastening assembly for adjustable tracheotomy tube in Fig. 1, showing that the tab is pivoted;
Fig. 7 is an operational view of the fastening assembly for adjustable tracheotomy tube in Fig. 1, showing a tracheotomy tube is inserted into the trachea of a patient;
Fig. 8 is a perspective view of a second embodiment of a fastening assembly for adjustable tracheotomy tube in accordance with the present invention;
Fig. 9 is an exploded perspective view of the fastening assembly for adjustable tracheotomy tube in Fig. 8;
Fig. 10 is an enlarged perspective view of the tab in Fig. 8;
Fig. 11 is a cross sectional top view of the fastening assembly for adjustable tracheotomy tube in Fig. 8 showing that the tab is pivoted;
Fig. 12 is a cross sectional top view of the fastening assembly for adjustable tracheotomy tube in Fig. 8, showing that the tab is pulled back;
Fig. 13 is a perspective view of a conventional fastening assembly for adjustable tracheotomy tube in accordance with the prior art; and
Fig. 14 is an enlarged side view in partial section of the conventional fastening assembly for adjustable tracheotomy tube in Fig. 13.

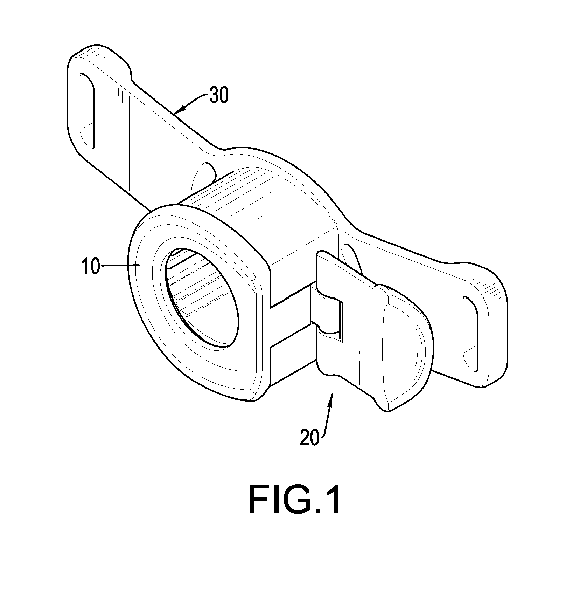

With reference to Figs. 1 to 3, a first embodiment of a fastening assembly for adjustable tracheotomy tube in accordance with the present invention comprises a plug 10, a clamping member 20 and a connector 30.

The plug 10 has a flange 11 and a tube section 12. The flange 11 has a side, a central area, and a plug hole 110. The plug hole 110 is formed through the central area of the flange 11.

The tube section 12 is integrally connected with the side of the flange 11 and has an outer surface, an end surface, a plug space 120, a flat area 121, a curb 122 and a plug opening 123.

The end surface of the tube section 12 is opposite to the flange 11.

The plug space 120 is formed within the tube section 12 and communicates with the plug hole 110.

The flat area 121 is formed on the outer surface of the tube section 12 and has a central portion.

The curb 122 protrudes from the central portion of the flat area 121 and is connected with the flange 11.

The plug opening 123 is formed in the end surface of the tube section 12, is formed through the flat area 121 beside the curb 122 and communicates with the plug space 120.

With reference to Figs. 1 to 3, the clamping member 20 is mounted in the plug 10 and has a sleeve 21, a connecting section 22 and a tab 23.

The sleeve 21 is slidably mounted in the plug space 120 to selectively align with the plug hole 110, and has an inner surface 211 and an outer surface. Preferably, the inner surface 211 of the sleeve 21 is jagged. The outer surface of the sleeve 21 has an abutted area 212.

The connecting section 22 is securely disposed at the abutted area 212 of the outer surface of the sleeve 21 and is mounted through the plug opening 123. The connecting section 22 has two side surfaces, a protuberance 221, a hump 222, a flip 223 and two lump recesses 224.

The protuberance 221 protrudes from the abutted area 212 of the outer surface of the sleeve 21.

The hump 222 is integrally connected with the protuberance 221, is opposite to the sleeve 21, and has a hump corner 2221 adjacent to the protuberance 221.

The flip 223 is integrally connected with the abutted area 212 of the sleeve 21, the protuberance 221 and the hump 222 beside the hump corner 2221.

The lump recesses 224 are respectively formed in the side surfaces of the connecting section 22, are respectively located at two opposite sides of the hump 222.

The tab 23 is pivotally connected with the connecting section 22. The tab 23 has an end surface, a tab opening 231, two inner surfaces, two pivoting lumps 232 and a tap bump 233.

The tab opening 231 is rectangular and is formed in the end surface of the tab 23.

The inner surfaces of the tab 23 are formed in the tab opening 231 and are opposite to each other.

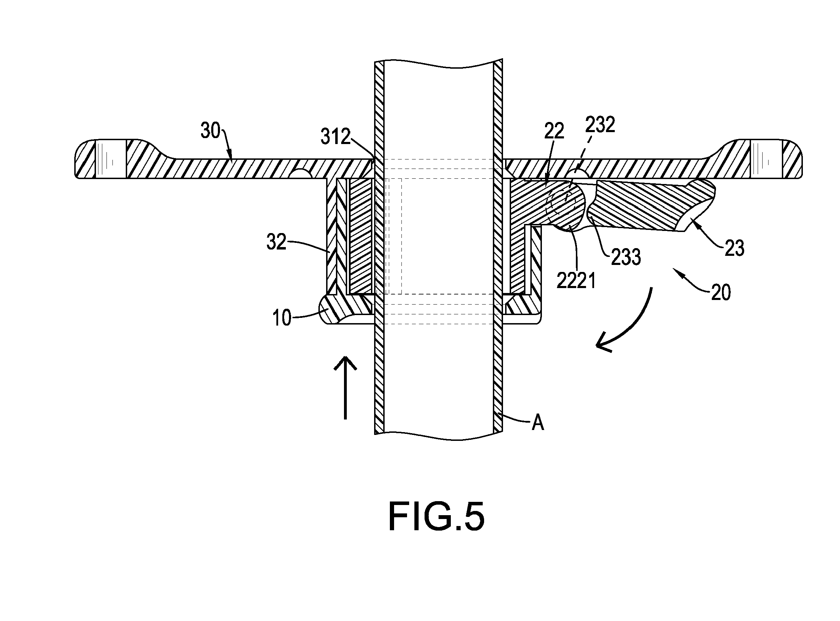

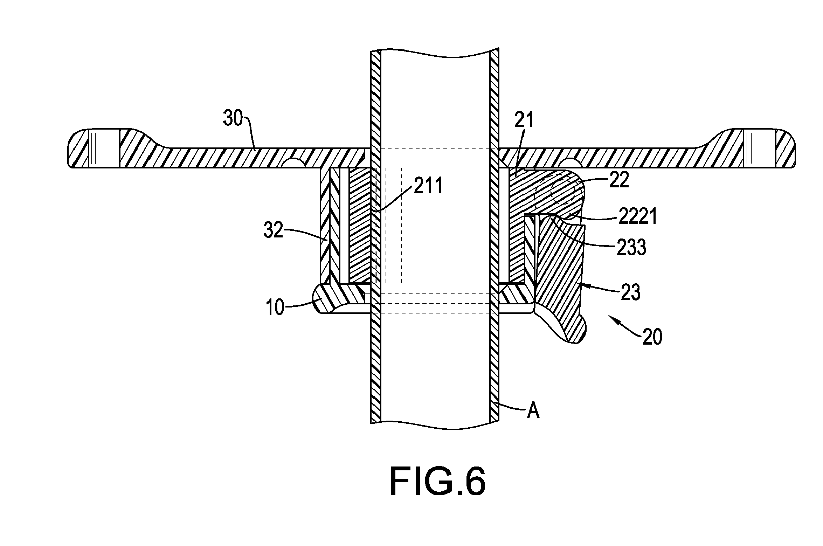

The pivoting lumps 232 respectively protrude from the inner surfaces of the tab 23, and are respectively and rotatably inserted into the lump recesses 224. With reference to Figs. 5 and 6, each pivoting lump 232 is eccentric relative to a corresponding one of the inner surfaces of the tab 23.

The tab bump 233 protrudes from the tab 23 toward the connecting section 22, is located in the tab opening 231 beside the pivoting lumps 232, and is capable of being slid over the hump corner 2221 and being held by the hump corner 2221 and the protuberance 221.

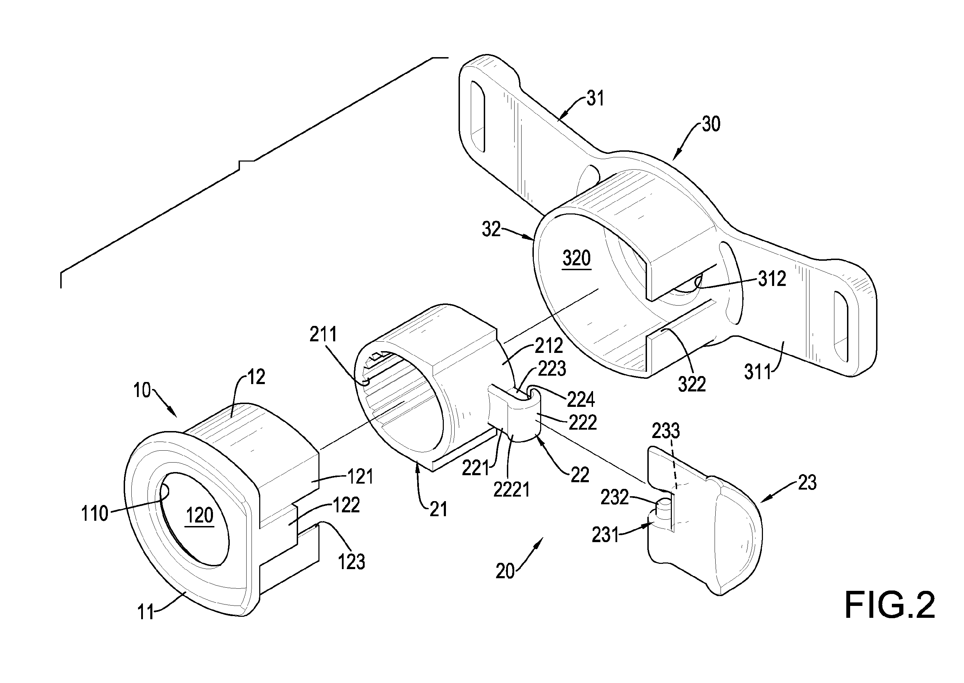

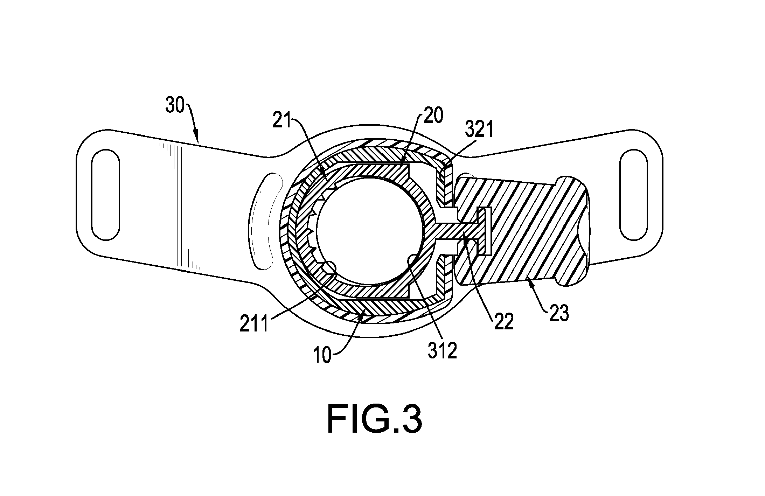

With reference to Figs. 2 and 3, the connector 30 encompasses the plug 10 and the clamping member 20, and has a base 31 and a hoop 32.

The base 31 has a central section, two neck plates 311 and a base hole 312. The neck plates 311 are integrally and respectively connected with two opposite sides of the central section of the base 31. The base hole 312 is formed through the central section of the base 31 and aligns with the plug hole 110.

The hoop 32 is integrally connected with the central section of the base 31, is capable of being tightly pressed against by the tab 23 such that the sleeve 21 can be slid in the plug space 120. The hoop 32 has an outer surface, a hoop space 320, an inner surface, a flat area 321 and a trench 322.

The hoop space 320 is formed within the hoop 32 and encompasses the tube section 12.

The flat area 321 of the hoop 32 is formed on the inner surface of the hoop 32 and is abutted by the flat area 121 of the tube section 12.

The trench 322 is formed in the outer surface of the hoop 32 and communicates with the hoop space 320. The connecting section 22 is mounted through the trench 322 and the tab 23 is located outside the hoop 32. The curb 122 is mounted in the trench 322.

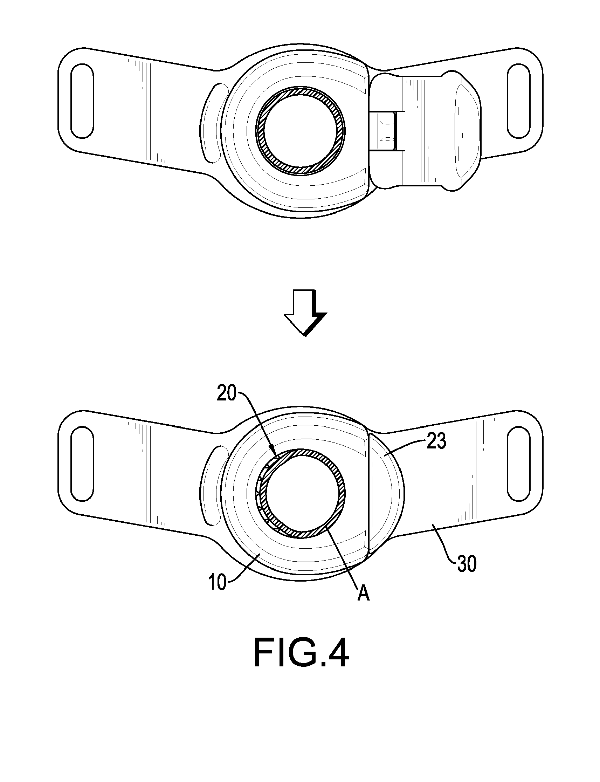

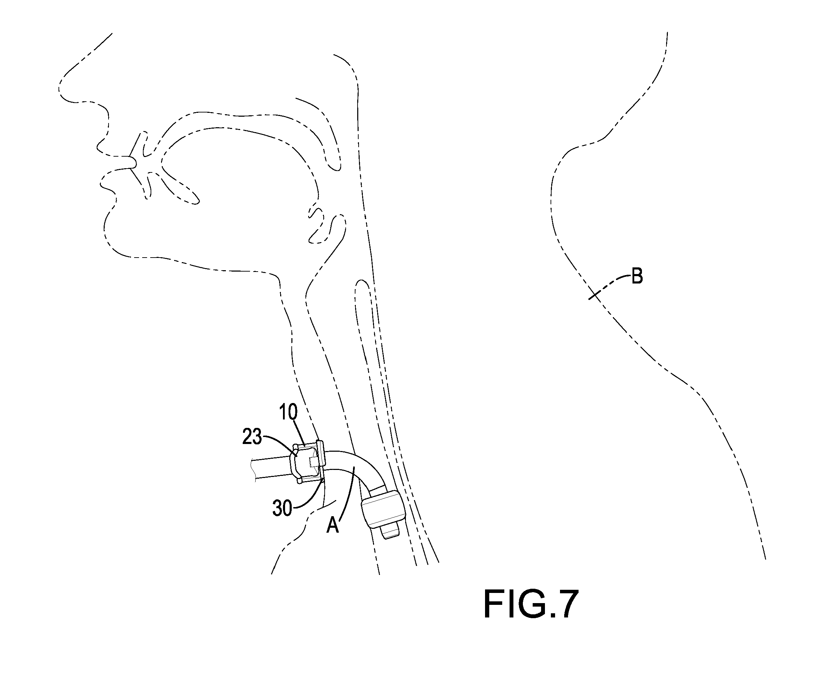

With reference to Figs. 4 to 7, a tracheotomy tube A is mounted through the plug 10, the clamping member 20 and the connector 30, and the tracheotomy tube A is about to be fastened by the fastening assembly for adjustable tracheotomy tube in accordance with the present invention.

First, the tracheotomy tube A is inserted into a trachea of a patient B, and is adjusted to a proper position.

Second, the tab 23 is pivoted toward the hoop 32. The sleeve 21 is slid to abut the flat area 321 of the inner surface of the hoop 32 when the tab bump 233 is slid over the hump corner 2221. When the tab bump 233 is held by the hump corner 2221 and the protuberance 221, the tracheotomy tube A is fastened by the sleeve 20 with the inner surface 211 of the sleeve 20 abutting the tracheotomy tube A.

When the tracheotomy tube A is about to be pulled out of the trachea of the patient B, the tab 23 is pivoted backward to return to an original position. Accordingly, the tracheotomy tube A can be moved and pulled out.

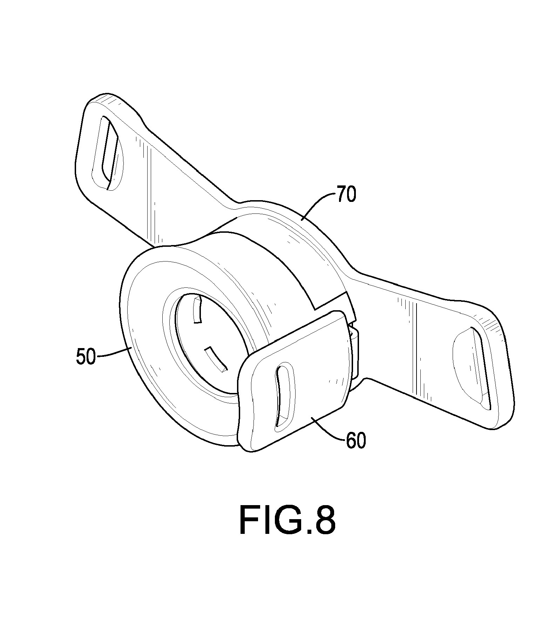

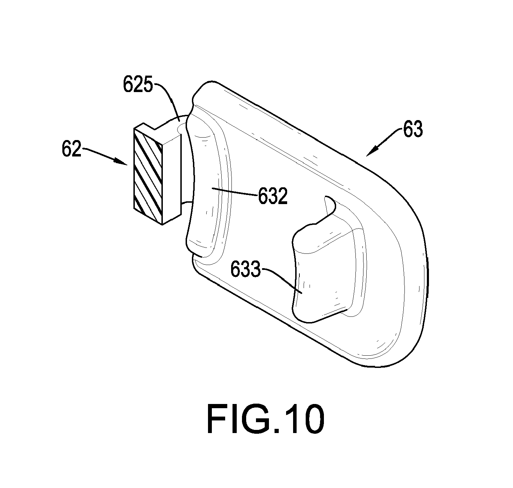

With reference to Figs. 8 to 12, a second embodiment of the fastening assembly for adjustable tracheotomy tube is substantially the same as the first embodiment and has a plug 50, a clamping member 60 and a connector 70.

The plug 50 has a flange 51 and a tube section 52. The flange 51 has a plug hole 510 and a chamfer 511. The chamfer 511 is annular and is formed in a side of the flange 51 opposite to the tube section 52. The tube section 52 of the plug 50 has a plug space 520, a plug opening 523, a furrow 524, two chunks 525 and a groove 526. The furrow 524 is C-shaped and is formed in the end surface of the tube section 52 beside the plug opening 523.

The chunks 525 are located between the furrow 524 and the plug opening 523, are respectively next to the plug opening 523, are located in the trench 722, and respectively abut two inner side surfaces of the trench 722.

The groove 526 is C-shaped, is formed in the tube section 52 and is located around the furrow 524.

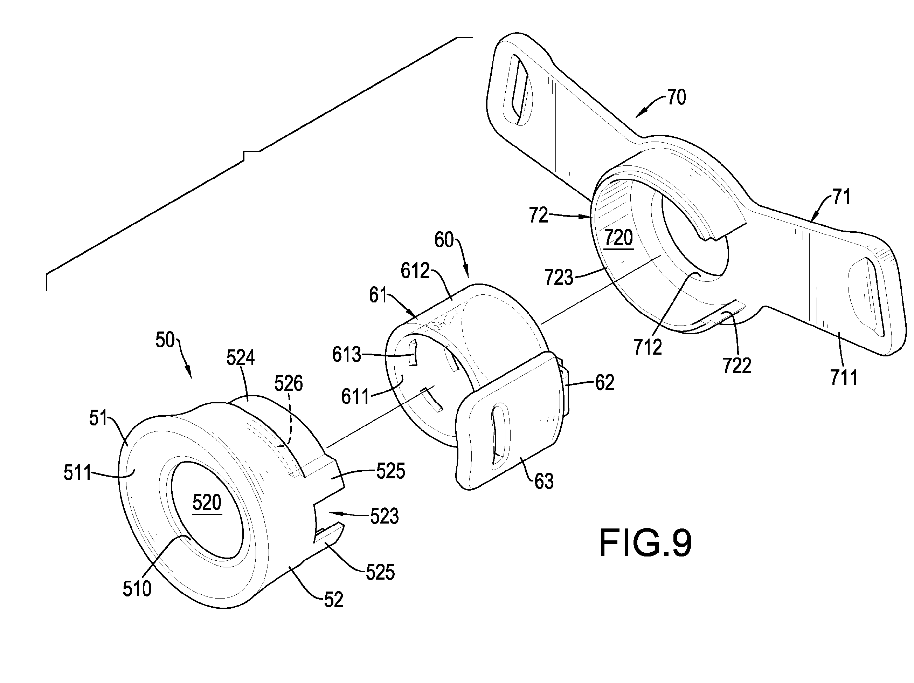

With reference to Figs. 9 to 11, the clamping member 60 is made of plastics and has a sleeve 61, a connecting section 62 and a tab 63.

The sleeve 61 has an inner surface 611, an outer surface 612 and multiple teeth 613. The teeth 613 protrude from the inner surface 611 of the sleeve 61. Each tooth 613 extends along an inner periphery of the sleeve 61.

The connecting section 62 is flexible, is integrally connected with the outer surface 612 of the sleeve 61 and has a flexible portion 625 opposite to the sleeve 61.

The tab 63 is integrally connected with the flexible portion 625 and has an inner side surface, a protrusion 632 and a tab bump 633.

The inner side surface of the tab 63 faces the plug 50. The protrusion 632 is formed on the inner side surface of the tab 63 and is adjacent to the connecting section 62.

The tab bump 633 protrudes from the inner side surface of the tab 63 beside the protrusion 632. The tab bump 633 is capable of being slid over a rim of the chamfer 511 of the flange 51 and being held by the rim of the chamfer 511 of the flange 51, and meanwhile the protrusion 632 serves as a fulcrum for moving the sleeve 61 in the hoop space 720 when the tab 63 is pivoted on the connecting section 62.

The connector 70 has a base 71 and a hoop 72. The base 71 has two neck plates 711 and a base hole 712.

The hoop 72 has an end surface and a rib 723. The rib 723 is C-shaped, protrudes from the end surface of the hoop 72 and is detachably inserted into the groove 526, such that the plug 50 is connected with the connector 70 without relative rotation therebetween.

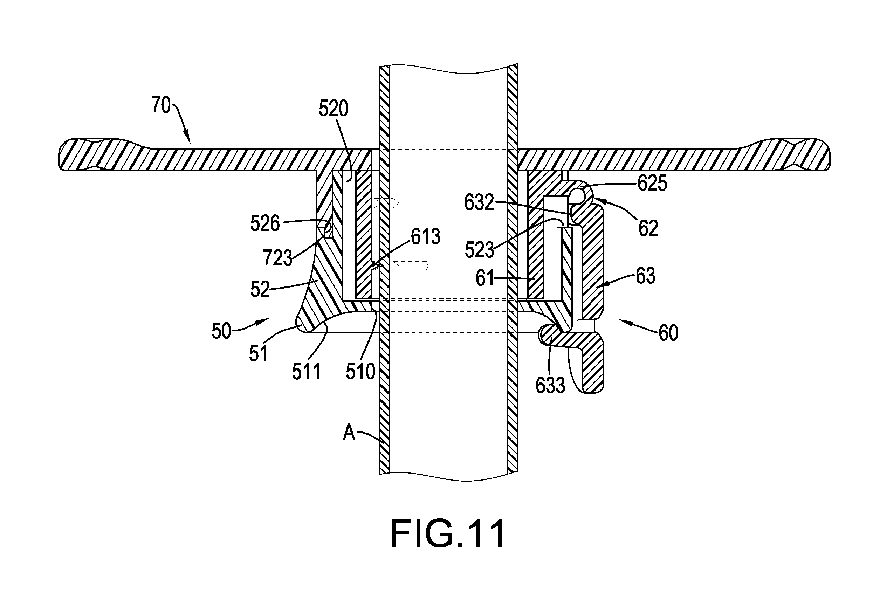

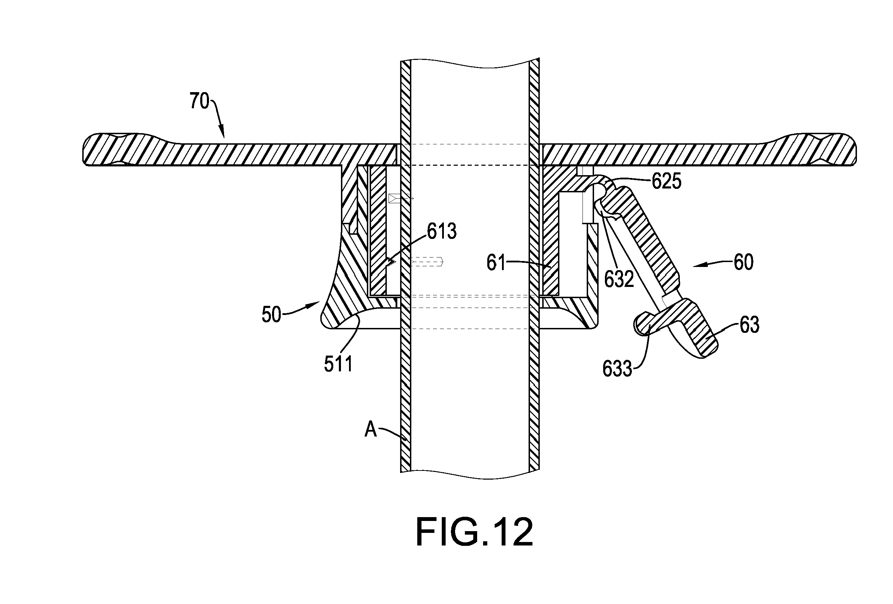

With reference to Figs. 11 and 12, the tracheotomy tube A is about to be fastened by the second embodiment of the fastening assembly for adjustable tracheotomy tube. The tab 63 is pivoted, and then the protrusion 632 abuts the plug 50 to serve as a fulcrum. The sleeve 61 is slid in the hoop space 720 as the tap bump 633 is slid over the rim of the chamfer 511 of the flange 51. Accordingly, the teeth 613 are pressed against the tracheotomy tube A tightly to prevent the tracheotomy tube A from being moved.

From the above description, it is noted that the present invention has the following advantage:

It is very convenient for a user, especially for taking care of a patient, to pivot the tab 23, 63 with one hand. Accordingly, the tracheotomy tube A is fastened with ease.

Even though numerous characteristics and advantages of the present invention have been set forth in the foregoing description, together with details of the structure and function of the invention, the disclosure is illustrative only, and changes may be made in detail, especially in matters of shape, size, and arrangement of parts within the principles of the invention to the full extent indicated by the broad general meaning of the terms in which the appended claims are expressed.

## Claims

1. A fastening assembly for adjustable tracheotomy tube, **characterized in that** the fastening assembly comprising:
a plug (10,50) having
a flange (11,51) having
a side;
a central area; and
a plug hole (110,510) formed through the central area of the flange (11,51);
a tube section (12,52) integrally connected with the side of the flange (11,51) and having
an end surface opposite to the flange (11,51)
a plug space (120,520) formed within the tube section (12,52) and communicating with the plug hole (110,510); and
a plug opening (123,523) formed in the end surface of the tube section (12,52) and communicating with the plug space (120,520);
a clamping member (20,60) mounted in the plug (10,50) and having
a sleeve (21,61) slidably mounted in the plug space (120,520) to selectively align with the plug hole (110,510), and having an outer surface (612);
a connecting section (22,62) securely disposed at the outer surface of the sleeve (21,61) and mounted through the plug opening (123,523); and
a tab (23,63) pivotally connected with the connecting section (22,62); and
a connector (30,70) encompassing the plug (10,50) and the clamping member (20,60), and having
a hoop (32,72) capable of being tightly pressed against by the tab (23,63) such that the sleeve (21,61) can be slid in the plug space (120,520), the hoop (32,72) having
an outer surface;
an inner surface;
a hoop space (320,720) formed within the hoop (32,72) and encompassing the tube section (12,52); and
a trench (322,722) formed in the outer surface of the hoop (32,72) and communicating with the hoop space (320,720), wherein the connecting section (22,62) is mounted through the trench (322,722).

2. The fastening assembly for adjustable tracheotomy tube as claimed in claim 1, wherein
the connecting section (22) has
two side surfaces; and
two lump recesses (224) respectively formed in the side surfaces of the connecting section (22); and
the tab (23) has
an end surface;
a rectangular tab opening (231) formed in the end surface of the tab (23);
two opposite inner surfaces formed in the tab opening (231);
two pivoting lumps (232) respectively protruding from the inner surfaces of the tab (23), and respectively and rotatably inserted into the lump recesses (224), wherein each pivoting lump (232) is eccentric relative to a corresponding one of the inner surfaces of the tab (23).

3. The fastening assembly for adjustable tracheotomy tube as claimed in claim 2, wherein
the connecting section (22) has
a protuberance (221) protruding from the outer surface of the sleeve (21); and
a hump (222) connected with the protuberance (221), opposite to the sleeve (21), and having a hump corner (2221) adjacent to the protuberance (221); and
the tab (23) has
a tab bump (233) protruding from the tab (23) toward the connecting section (22), located in the tab opening (231) beside the pivoting lumps (232) and capable of being slid over the hump corner (2221) and being held by the hump corner (2221) and the protuberance (221).

4. The fastening assembly for adjustable tracheotomy tube as claimed in claim 3, wherein the sleeve (21) has a jagged inner surface (211).

5. The fastening assembly for adjustable tracheotomy tube as claimed in any one of claims 1 to 4, wherein
the tube section (12) has an outer surface having a flat area (121); and
the hoop (32) has an inner surface having a flat area (321) abutted by the flat area (121) of the tube section (12).

6. The fastening assembly for adjustable tracheotomy tube as claimed in claim 1, wherein
the tab (63) has
an inner side surface facing the plug (50);
a protrusion (632) formed on the inner side surface of the tab (63) and adjacent to the connecting section (62); and
a tab bump (633) protruding from the inner side surface of the tab (63) beside the protrusion (632), wherein the tab bump (633) is capable of being slid over a rim of the flange (51) and being held by the rim of the flange (51), and meanwhile the protrusion (632) serves as a fulcrum for moving the sleeve (61) in the hoop space (720) when the tab (63) is pivoted on the connecting section (62).

7. The fastening assembly for adjustable tracheotomy tube as claimed in claim 6, wherein the connecting section (62) is flexible and is integrally connected with the outer surface (612) of the sleeve (61) and the tab (63).

8. The fastening assembly for adjustable tracheotomy tube as claimed in claim 7, wherein the sleeve (61) has
an inner surface (611); and
multiple teeth (613) protruding from the inner surface (611) of the sleeve (61), each tooth (613) extending along an inner periphery of the sleeve (61).

9. The fastening assembly for adjustable tracheotomy tube as claimed in claim 6, 7 or 8, wherein
the tube section (52) of the plug (50) has
a C-shaped furrow (524) formed in the end surface of the tube section (52) beside the plug opening (523); and
two chunks (525) located between the furrow (524) and the plug opening (523), respectively next to the plug opening (523), located in the trench (722), and respectively abutting two inner side surfaces of the trench (722).

10. The fastening assembly for adjustable tracheotomy tube as claimed in claim 9, wherein
the sleeve (52) has a C-shaped groove (526) formed in the tube section (52); and
the hoop (72) has
an end surface; and
a C-shaped rib (723) protruding from the end surface of the hoop (72) and detachably inserted into the groove (526), such that the plug (50) is connected with the connector (70).
